# FASCICULE DE BREVET EUROPEEN

(11) **EP 0 684 035 B1**
(45) Date de publication et mention de la délivrance du brevet: **11.09.1996**
(21) Numéro de dépôt: 95400533.6
(22) Date de dépôt: 13.03.1995
(51) Int. Cl.: A61K 7/13

(54) **Composition de teinture d'oxydation des fibres kératiniques comprenant un dérivé de paraphénylènediamine et une 6-hydroxy 1,4-benzoxazine**
Keratinische Fasern Oxydationsfärbemittel enthaltend einen Derivaten von Paraphenylenediamine und einen 6-Hydroxy 1,4-Benzoxazine
Keratineous fibres oxidation dyeing composition containing a derivative of paraphenylenediamine and a 6-hydroxy 1,4-benzoxazine

(30) Priorité: 26.05.1994 FR 9406394
(43) Date de publication de la demande: 29.11.1995
(73) Titulaire: L'OREAL, F-75008 Paris (FR)
(72) Inventeur: Cotteret, Jean, F-78480 Verneuil s/Seine (FR); Audousset, Marie-Pascale, F-92600 Asnieres (FR)
(74) Mandataire: Andral, Christophe André Louis

(56) Documents cités:
- EP-A- 0 007 537
- FR-A- 2 015 589
- GB-A- 2 078 747

## Description

La présente invention concerne une composition de teinture d'oxydation des fibres kératiniques et en particulier des fibres kératiniques humaines, comprenant en association, au moins une paraphénylènediamine substituée en position 2 sur le noyau benzénique et une 6-hydroxy 1,4-benzoxazine, dont les structures sont données ci-après dans la description. Elle concerne également l'utilisation d'une telle composition.

Il est connu de teindre les fibres kératiniques et en particulier les cheveux humains, avec des compositions de teinture contenant des précurseurs de colorants d'oxydation, en particulier des ortho- ou paraphénylènediamines, des ortho-ou paraaminophénols, généralement appelés "bases d'oxydation", associés à des coupleurs encore appelés modificateurs de coloration, plus particulièrement des métaphénylènediamines, des méta-aminophénols et des métadiphénols, qui permettent de modifier et d'enrichir de reflets les colorations "de fond" obtenues par les produits de condensation des bases d'oxydation.

Ainsi, à la paraphénylènediamine, on associe traditionnellement le métadihydroxybenzène plus connu sous le nom de résorcine, pour obtenir des nuances naturelles intenses.

L'utilisation de la paraphénylènediamine étant remise en cause pour des raisons toxicologiques, on a déjà proposé dans la demande de brevet WO 80/0014 et dans le brevet EP-0 400 330 B1, d'utiliser en remplacement de la paraphénylènediamine, des dérivés de la paraphénylènediamine monohydroxyalkylés en position 2 sur le noyau benzénique.

Cependant, lorsqu'on associe de la résorcine à ces dérivés de paraphénylènediamine monohydroxyalkylés en position 2 sur le noyau benzénique, les nuances naturelles recherchées sont d'une intensité trop faible pour permettre au formulateur de créer une large palette de nuances.

Après d'importantes recherches menées sur la question, la Demanderesse vient maintenant de découvrir qu'il est possible d'obtenir de nouvelles teintures non toxiques, qui engendrent des nuances naturelles intenses, en associant une paraphénylènediamine substituée en position 2 sur le noyau benzénique et une 6-hydroxy 1,4-benzoxazine, de structures respectivement définies ci-après.

Cette découverte est à la base de la présente invention.

La présente invention a ainsi pour objet une composition de teinture d'oxydation pour fibres kératiniques, en particulier pour fibres kératiniques humaines, telles que les cheveux, du type comprenant, dans un milieu approprié pour la teinture, au moins un précurseur de colorant d'oxydation et au moins un coupleur, et qui est caractérisée par le fait qu'elle contient, à titre de précurseur de colorant d'oxydation, au moins une paraphénylènediamine de formule (I) suivante : 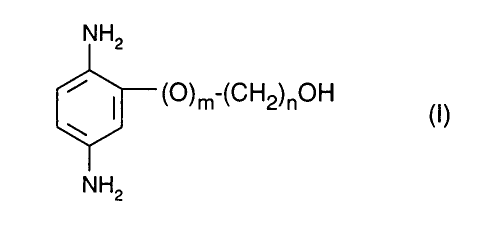
dans laquelle m est un nombre entier égal à zéro ou 1, et n est un nombre entier compris inclusivement entre 1 et 4,
et/ou au moins l'un des sels d'addition de cette paraphénylènediamine avec un acide,
et à titre de coupleur, au moins une 6-hydroxy 1,4-benzoxazine de formule (II) suivante : 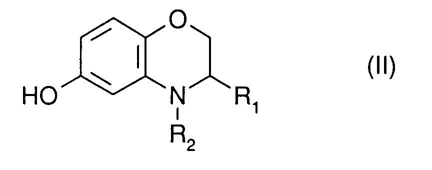
dans laquelle les radicaux R₁ et R₂ désignent, indépendamment l'un de l'autre ou simultanément, un atome d'hydrogène ou un radical alkyle inférieur contenant de 1 à 4 atomes de carbone,
et/ou au moins l'un des sels d'addition de cette benzoxazine avec un acide.

Les nouvelles teintures ainsi obtenues permettent d'aboutir à des colorations naturelles soutenues et non toxiques.

Ces nouvelles teintures présentent en outre une bonne résistance aux shampooings et permettent d'obtenir une bonne couverture des cheveux blancs.

Un autre objet de l'invention porte sur la composition prête à l'emploi, contenant les différents agents utilisés pour la teinture des fibres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, au moins un précurseur de colorant d'oxydation et au moins un coupleur tels qu'ils ont été définis ci-avant, la couleur étant révélée à pH alcalin neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture, ou "kits" à plusieurs compartiments, dont le premier compartiment contient au moins une paraphénylènediamine de formule (I), à titre de précurseur de colorant d'oxydation, et au moins une 6-hydroxy 1,4-benzoxazine de formule (II), à titre de coupleur, et le deuxième compartiment un agent oxydant.

D'autres caractéristiques, aspects, objets et avantages de l'invention apparaîtront encore plus clairement à la lecture de la description et des exemples qui suivent.

Les sels d'acide qui peuvent être utilisés selon l'invention sont choisis de préférence parmi les chlorhydrates, les sulfates, les bromhydrates et les tartrates.

Parmi les précurseurs de colorant d'oxydation utilisables dans le cadre de la présente invention, on préfère mettre en oeuvre les composés suivants : la 2-hydroxyméthyl paraphénylènediamine, la 2-β-hydroxyéthyl paraphénylènediamine, et la 2-β-hydroxyéthyloxy paraphénylènediamine. Encore plus préférentiellement, on met en oeuvre la 2-β-hydroxyéthyl paraphénylènediamine.

La concentration en ce(s) précurseurs(s) ou leurs sels peut varier entre 0,01 et 10 % en poids environ par rapport au poids total de la composition de teinture et de préférence entre 0,05 et 5 % en poids environ.

Parmi les coupleurs de formule (II), on préfère plus particulièrement mettre en oeuvre la 6-hydroxy 1,4-benzoxazine et la N-méthyl 6-hydroxy 1,4-benzoxazine.

La concentration en coupleur(s) de formule (II) ou leurs sels peut varier entre 0,005 et 5% en poids environ par rapport au poids total de la composition de teinture et de préférence entre 0,01 et 3% en poids environ.

Des compositions de teinture d'oxydation plus particulièrement préférées selon l'invention comprennent à titre de précurseur de colorant d'oxydation la 2-β-hydroxyéthyl paraphénylènediamine ou l'un de ses sels, et à titre de coupleur la 6-hydroxy 1,4-benzoxazine ou la N-méthyl 6-hydroxy 1,4-benzoxazine ou l'un de leurs sels.

D'autres compositions, également particulièrement intéressantes, comprennent à titre de précurseur de colorant d'oxydation la 2-β-hydroxyéthyloxy paraphénylènediamine ou l'un de ses sels, et à titre de coupleur, la 6-hydroxy 1,4-benzoxazine ou la N-méthyl 6-hydroxy 1,4-benzoxazine ou l'un de leurs sels.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie au moyen soit d'agents alcalinisants habituellement utilisés en teinture des fibres kératiniques, tels que l'ammoniaque, les carbonates alcalins, les alcanolamines, par exemple les mono-, di- et triéthanolamines ainsi que leurs dérivés, les hydroxydes de sodium ou de potassium, les composés de formule : 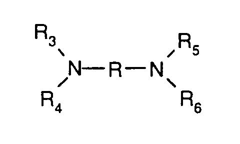
dans laquelle R est un reste propylène éventuellement substitué par un groupement hydroxyle ou un radical alkyle en C₁-C₄ ; R₃, R₄, R₅ et R₆, simultanément ou indépendamment l'un de l'autre, représentent un atome d'hydrogène, un radical alkyle en C₁-C₄ ou hydroxyalkyle en C₁-C₄,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus est tel qu'après un mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et d'autres bases d'oxydation de formules (I) et (II) et/ou des colorants directs et/ou des précurseurs de mélanine, notamment pour modifier les nuances ou les enrichir en reflets. Dans ce contexte, on peut citer tout particulièrement l'association de la 2-β-hydroxyéthyl paraphénylènediamine, de la 6-hydroxy 1,4-benzoxazine et du 6-hydroxyindole, ou de leurs sels d'addition avec un acide.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tert.butyl hydroquinone, la 3-méthyl 1-phényl 5-pyrazolone et l'acide homogentisique et sont alors généralement présents dans des proportions comprises entre environ 0,05 et 1,5 % en poids par rapport au poids total de la composition.

Les compositions de teinture contiennent également, dans leur forme de réalisation préférée, des agents tensioactifs bien connus de la technique, dans des proportions comprises entre environ 0,5 et 55 % en poids, et de préférence entre 2 et 50 % en poids par rapport au poids total de la composition, des solvants organiques, dans des proportions comprises entre environ 1 et 40 % en poids, et en particulier entre 5 et 30 % en poids par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés.

### EXEMPLES

### Exemple 1

On a prépare la composition de teinture, conforme à l'invention, suivante :

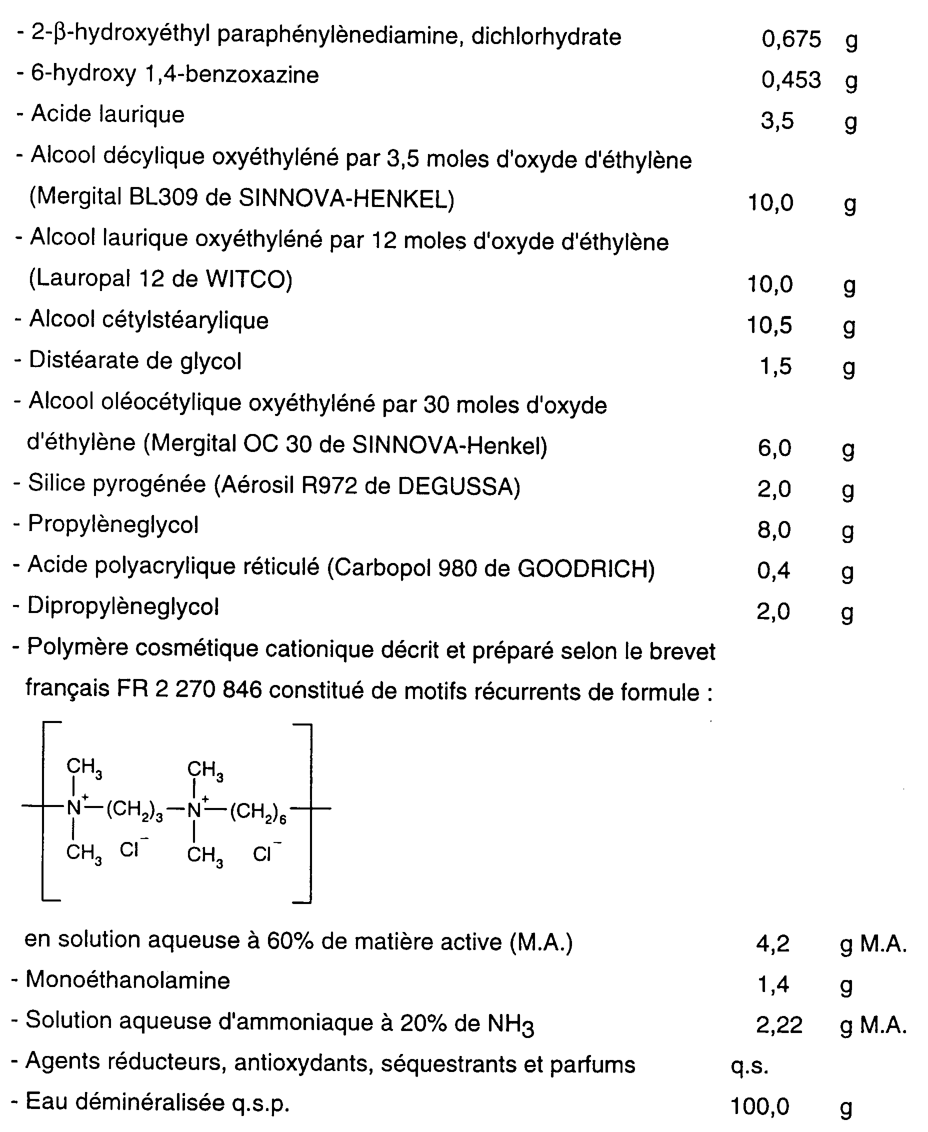

Au moment de l'emploi, on a mélangé cette composition avec 1,5 fois son poids en eau oxygénée titrant 20 volumes (6% en poids), de pH 3.
On a obtenu un mélange de pH 9,8.

On a ensuite appliqué ce mélange sur deux qualités de cheveux: des cheveux gris à 90% de blancs, des cheveux gris à 90% de blancs permanentés, pendant 30 minutes. Après rinçage, lavage au shampooing, rinçage et séchage, les cheveux sont teints dans une nuance d'une intensité bien plus soutenue que celle obtenue à partir d'une composition de teinture identique mais dans laquelle on remplace la 6-hydroxy 1,4-benzoxazine par une quantité molaire équivalente de résorcine (0,327 g).

Ces nuances comparées sont mesurées en valeurs L, a, b (système de notation de la couleur dans lequel L désigne l'intensité, a désigne la nuance, et b désigne la pureté) sur un colorimètre MINOLTA CM2002.

Les valeurs de l'intensité de la coloration chiffrées en L sont les suivantes :

| Valeur de L | sur cheveux 90% blancs | sur cheveux 90% blancs permanentés |
|---|---|---|
| Composition de teinture conforme à l'invention | 43,19 | 32,82 |
| Contretype à la résorcine | 47,40 | 38,78 |

Dans le système de notation L, a, b, plus la valeur de L est basse, plus la nuance est intense.

On constate donc bien une nuance significativement plus intense avec la composition selon l'invention, comparativement à celle obtenue avec son contretype de l'art antérieur à la résorcine.

### Exemple 2 :

On a préparé la composition de teinture, conforme à l'invention, suivante :

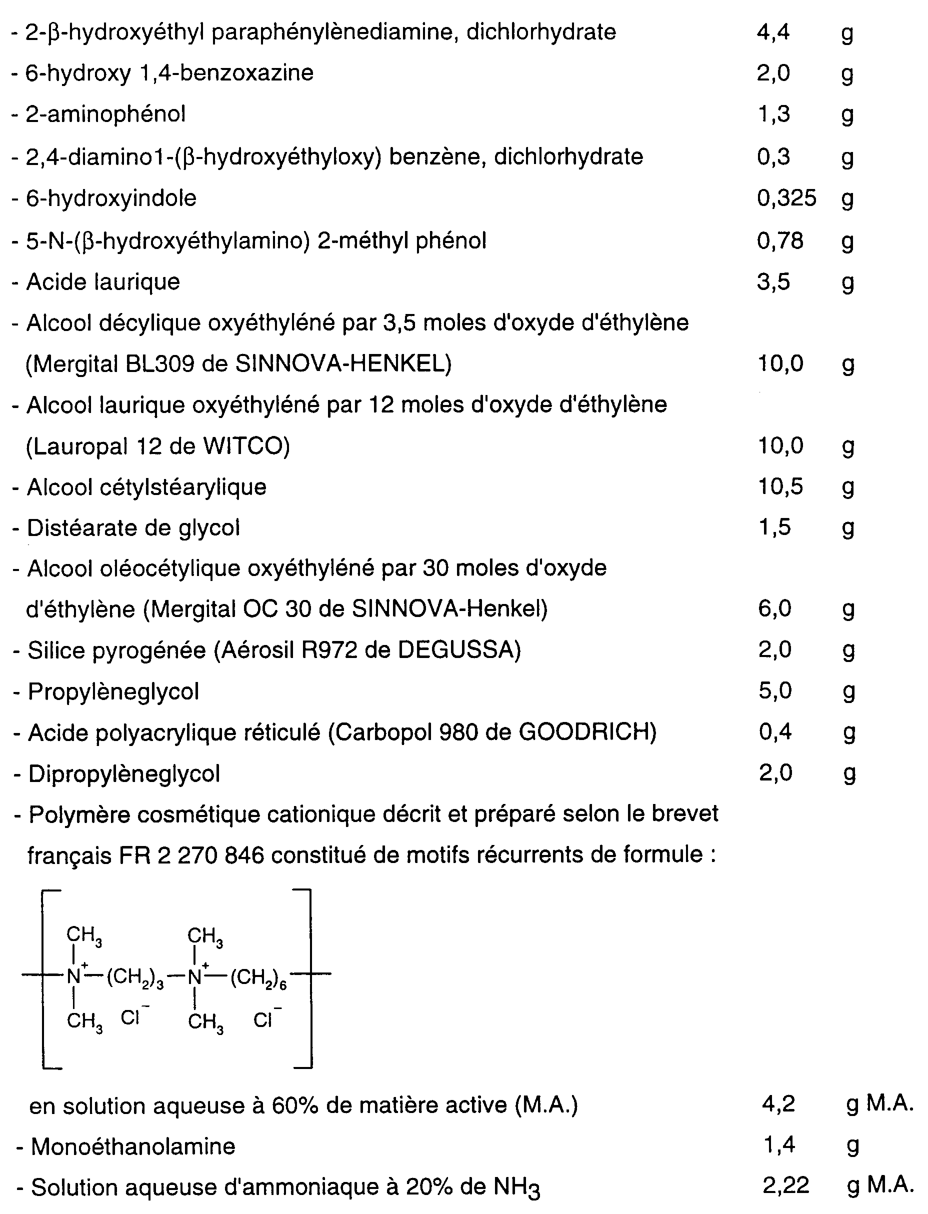

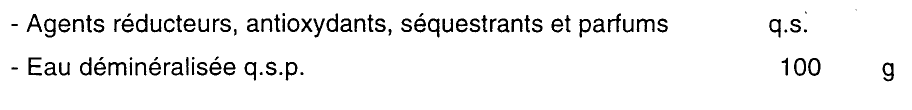

En suivant le même protocole de teinture qu'à l'exemple 1, on obtient une nuance châtain particulièrement intense sur cheveux naturels gris à 90% de blancs ainsi que sur cheveux permanentés gris à 90% de blancs.

### Exemple 3 :

On a préparé la composition de teinture, conforme à l'invention, suivante :

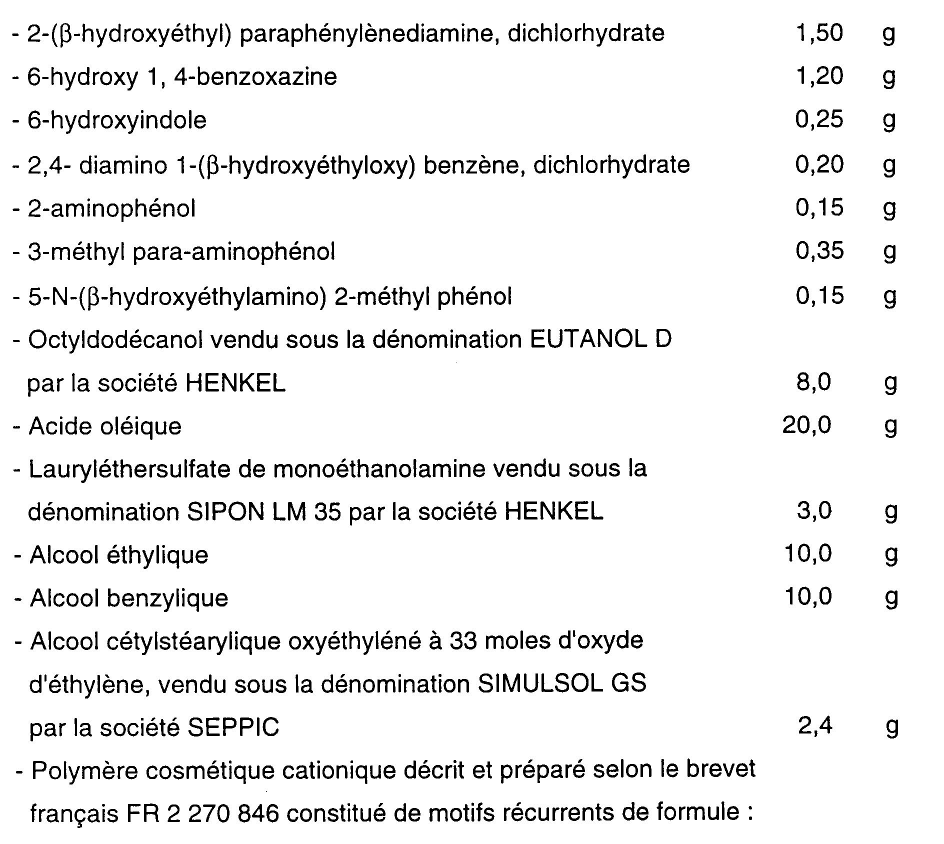

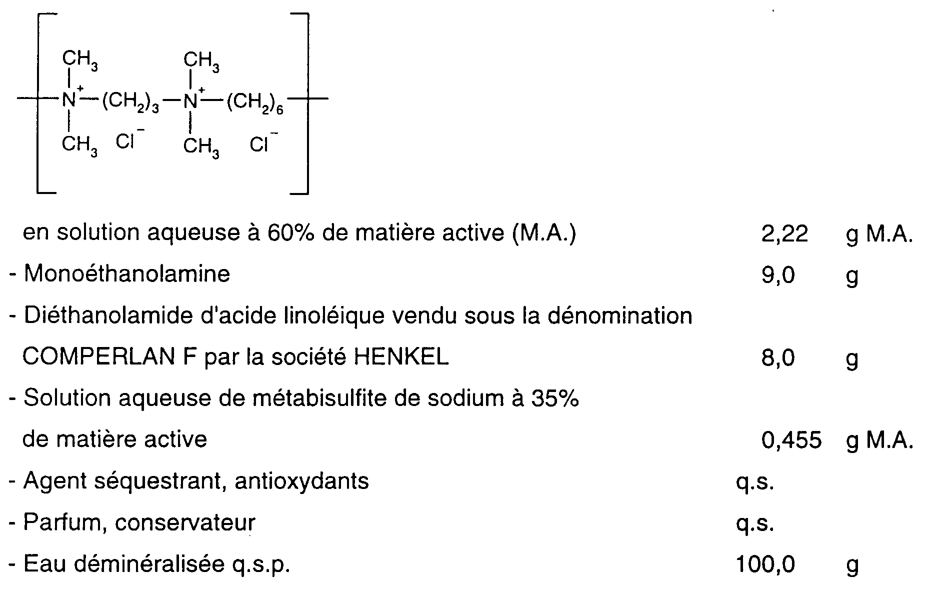

Au moment de l'emploi, on a mélangé cette composition avec 2 fois son poids en eau oxygénée titrant 9 volumes, de pH 3.
On a obtenu un mélange de pH 9,2.

On a ensuite appliqué ce mélange sur des cheveux gris permanentés et non permanentés à 90% de blancs, pendant 15 minutes. Après rinçage, lavage au shampooing, rinçage et séchage, les cheveux sont teints dans une nuance blond foncé intense.

## Revendications

1. Composition de teinture d'oxydation pour fibres kératiniques, en particulier pour fibres kératiniques humaines telles que les cheveux, du type comprenant dans un milieu approprié pour la teinture, au moins un précurseur de colorant d'oxydation et au moins un coupleur, caractérisée par le fait qu'elle contient, à titre de précurseur de colorant d'oxydation, au moins une paraphénylènediamine de formule (I) suivante : 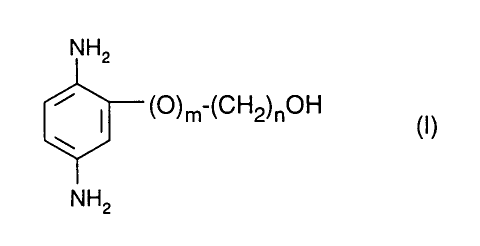 dans laquelle m est un nombre entier égal à zéro ou 1, et n est un nombre entier compris inclusivement entre 1 et 4,
et/ou au moins l'un des sels d'addition de cette paraphénylènediamine avec un acide,
et à titre de coupleur, au moins une 6-hydroxy 1,4-benzoxazine de formule (II) suivante : 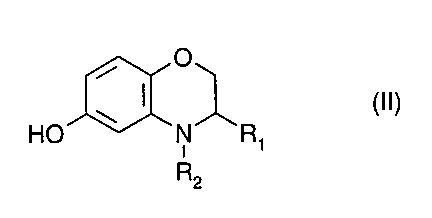 dans laquelle les radicaux R₁ et R₂ désignent, indépendamment l'un de l'autre ou simultanément, un atome d'hydrogène ou un radical alkyle inférieur contenant de 1 à 4 atomes de carbone,
et/ou au moins l'un des sels d'addition de cette benzoxazine avec un acide.

2. Composition de teinture selon la revendication 1, caractérisée par le fait que la paraphénylènediamine de formule (I) est choisie parmi la 2-hydroxyméthyl paraphénylènediamine, la 2-β-hydroxyéthyl paraphénylènediamine et la 2-β-hydroxyéthyloxy paraphénylènediamine, ou leurs sels d'addition avec un acide.

3. Composition de teinture selon la revendication 1 ou 2, caractérisée par le fait que le coupleur de formule (II) est choisi parmi la 6-hydroxy 1,4-benzoxazine ou la N-méthyl 6-hydroxy 1,4-benzoxazine.

4. Composition de teinture selon l'une quelconque des revendications 1 à 3, caractérisée par le fait qu'elle contient au moins la 2-β-hydroxyéthyl paraphénylènediamine à titre de précurseur de colorant d'oxydation de formule (I) et au moins la 6-hydroxy 1,4-benzoxazine ou la N-méthyl 6-hydroxy 1,4-benzoxazine à titre de coupleur de formule (II), ou leurs sels d'addition avec un acide.

5. Composition de teinture selon l'une quelconque des revendications 1 à 3, caractérisée par le fait qu'elle contient au moins la 2-β-hydroxyéthyloxy paraphénylènediamine à titre de précurseur de colorant d'oxydation de formule (I) et au moins la 6-hydroxy 1,4-benzoxazine ou la N-méthyl 6-hydroxy 1,4-benzoxazine à titre de coupleur de formule (II), ou leurs sels d'addition avec un acide.

6. Composition de teinture selon la revendication 1, caractérisée par le fait qu'elle contient au moins à titre de précurseur de colorant d'oxydation de formule (I), la 2-β-hydroxyéthyl paraphénylènediamine, à titre de coupleur la 6-hydroxy 1,4-benzoxazine, et à titre de colorant additionnel, du 6-hydroxyindole, ou leurs sels d'addition avec un acide.

7. Composition de teinture selon l'une quelconque des revendications 1 à 6, caractérisée par le fait que les sels d'addition avec un acide sont choisis parmi les chlorhydrates, les sulfates, les bromhydrates et les tartrates.

8. Composition de teinture selon l'une quelconque des revendications 1 à 7, caractérisée par le fait que la paraphénylènediamine de formule (I), ou ses sels, est présente dans une concentration comprise entre 0,01 et 10 % en poids par rapport au poids total de la composition, et de préférence entre 0,05 et 5 % en poids, et que le coupleur de formule (II), ou ses sels, est présent dans une concentration comprise entre 0,005 et 5 % en poids et de préférence entre 0,01 et 3 % par rapport au poids total de la composition.

9. Composition de teinture selon l'une quelconque des revendications 1 à 8, prête à l'emploi, caractérisée en ce qu'elle contient en outre un agent oxydant et qu'elle possède un pH compris entre 3 et 11.

10. Procédé de teinture des fibres kératiniques et en particulier des fibres kératiniques humaines telles que les cheveux, caractérisé par le fait qu'il consiste à appliquer sur les fibres une composition de teinture (A) selon l'une quelconque des revendications 1 à 9, et à révéler la couleur en milieu alcalin neutre ou acide à l'aide d'un agent oxydant qui est ajouté juste au moment de l'emploi à cette composition (A) ou qui est présent dans une composition (B) appliquée simultanément ou séquentiellement de façon séparée.

11. Dispositif à plusieurs compartiments, ou "Kit" pour la teinture des fibres kératiniques et en particulier des fibres kératiniques humaines telles que les cheveux, caractérisé par le fait qu'il comporte au moins deux compartiments, dont l'un d'entre eux renferme une composition (A) telle que définie dans les revendications 1 à 8, et un autre une composition (B) comprenant un agent oxydant dans un milieu approprié pour la teinture.

12. Utilisation d'une composition de teinture selon les revendications 1 à 9 ou d'un dispositif de teinture ou "Kit" à plusieurs compartiments selon la revendication 11, pour la teinture des fibres kératiniques humaines telles que les cheveux.

## Claims

1. Oxidation dyeing composition for keratinous fibres, in particular for human keratinous fibres such as hair, of the type which comprises, in a medium appropriate for dyeing, at least one oxidation dye precursor and at least one coupler, characterized in that it contains, as oxidation dye precursor, at least one para-phenylenediamine of the following formula (I): 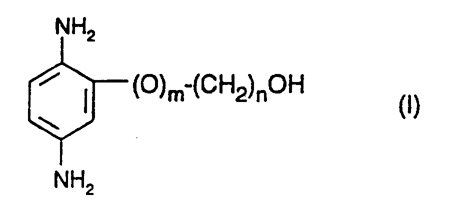 in which m is an integer equal to zero or 1, and n is an integer between 1 and 4 inclusive,
and/or at least one of the addition salts of this paraphenylenediamine with an acid,
and, as coupling agent, at least one 6-hydroxy-1,4-benzoxazine of the following formula (II): 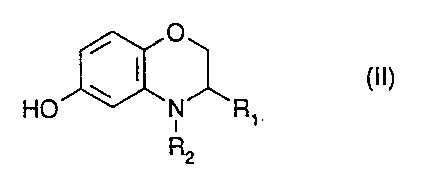 in which the radicals R₁ and R₂ denote, independently of one another or simultaneously, a hydrogen atom or a lower alkyl radical containing 1 to 4 carbon atoms,
and/or at least one of the addition salts of this benzoxazine with an acid.

2. Dyeing composition according to Claim 1, characterized in that the para-phenylenediamine of formula (I) is chosen from 2-hydroxymethyl-para-phenylenediamine, 2-β-hydroxyethyl-para-phenylenediamine and 2-β-hydroxyethyloxy-para-phenylenediamine, or their addition salts with an acid.

3. Dyeing composition according to Claim 1 or 2, characterized in that the coupler of formula (II) is chosen from 6-hydroxy-1,4-benzoxazine and N-methyl-6-hydroxy-1,4-benzoxazine.

4. Dyeing composition according to any one of Claims 1 to 3, characterized in that it contains at least 2-β-hydroxyethyl-para-phenylenediamine as oxidation dye precursor of formula (I) and at least 6-hydroxy-1,4-benzoxazine or N-methyl-6-hydroxy-1,4-benzoxazine as coupler of formula (II), or their addition salts with an acid.

5. Dyeing composition according to any one of Claims 1 to 3, characterized in that it contains at least 2-β-hydroxyethyloxy-para-phenylenediamine as oxidation dye precursor of formula (I) and at least 6-hydroxy-1,4-benzoxazine or N-methyl-6-hydroxy-1,4-benzoxazine as coupler of formula (II), or their addition salts with an acid.

6. Dyeing composition according to Claim 1, characterized in that it contains at least, as oxidation dye precursor of formula (I), 2-β-hydroxy-ethyl-para-phenylenediamine, as coupler, 6-hydroxy-1,4-benzoxazine, and, as additional dye, 6-hydroxyindole, or their addition salts with an acid.

7. Dyeing composition according to any one of Claims 1 to 6, characterized in that the addition salts with an acid are chosen from hydrochlorides, sulphates, hydrobromides and tartrates.

8. Dyeing composition according to any one of Claims 1 to 7, characterized in that the para-phenylenediamine of formula (I) or its salts is or are present in a concentration of between 0.01 and 10 % by weight relative to the total weight of the composition, and preferably between 0.05 and 5 % by weight, and in that the coupler of formula (II) or its salts is or are present in a concentration of between 0.005 and 5 % by weight and, preferably, between 0.01 and 3 % relative to the total weight of the compositon.

9. Dyeing composition according to any one of Claims 1 to 8, which is ready to use, characterized in that it additionally contains an oxidizing agent and in that it has a pH of between 3 and 11.

10. Process for dyeing keratinous fibres and, in particular, human keratinous fibres such as hair, characterized in that it consists in applying to the fibres a dyeing composition (A) according to any one of Claims 1 to 9 and in revealing the colour in an alkaline, neutral or acid medium with the aid of an oxidizing agent which is added to this composition (A) at the time of use or which is present in a composition (B) which is applied simultaneously or sequentially and separately.

11. Multi-compartment device or kit for the dyeing of keratinous fibres and, in particular, human keratinous fibres such as hair, characterized in that it comprises at least two compartments, one of which contains a composition (A) as defined in Claims 1 to 8 and another of which contains a composition (B) comprising an oxidizing agent in a medium appropriate for dyeing.

12. Use of a dyeing composition according to Claims 1 to 9 or of a multi-compartment kit or dyeing device according to Claim 11 for the dyeing of human keratinous fibres such as hair.

## Patentansprüche

1. Zusammensetzung zum oxidativen Färben von Keratinfasern, insbesondere von menschlichen Keratinfasern, wie Haaren, die in einem zum Färben geeigneten Medium mindestens ein Farbstoffvorprodukt eines Oxidationsfarbstoffs und mindestens einen Kuppler enthält,
dadurch gekennzeichnet, daß
sie als Farbstoffvorprodukt der Oxidationsfarbe mindestens ein p-Phenylendiamin der folgenden Formel (I) 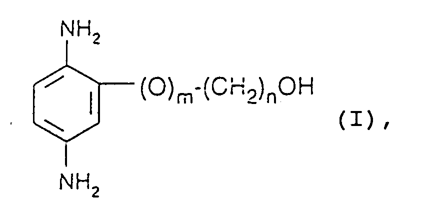 worin bedeuten:
m 0 oder 1 und
n eine ganze Zahl von 1 bis 4,
und/oder mindestens ein Additionssalz dieses p-Phenylendiamins mit einer Säure
und als Kuppler mindestens ein 6-Hydroxy-1,4-benzoxazin der folgenden Formel (II), 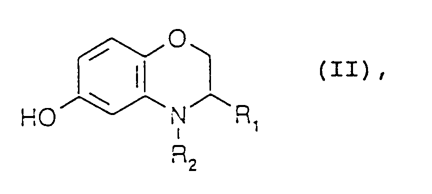 worin bedeuten:
R₁ und R₂, die gleich oder voneinander verschieden sind, ein Wasserstoffatom oder eine niedere Alkylgruppe mit 1 bis 4 Kohlenstoffatomen,
und/oder
mindestens ein Additionssalz dieses Benzoxazins mit einer Säure enthält.

2. Zusammensetzung zum Färben nach Anspruch 1, dadurch gekennzeichnet, daß das p-Phenylendiamin der Formel (I) unter 2-Hydroxymethyl-p-phenylendiamin, 2-(β-Hydroxyethyl)-p-phenylendiamin und 2-(β-Hydroxyethyloxy)-p-phenylendiamin oder den Additionssalzen dieser Verbindungen mit einer Säure ausgewählt ist.

3. Zusammensetzung zum Färben nach Anspruch 1 oder 2, dadurch gekennzeichnet, daß der Kuppler der Formel (II) unter 6-Hydroxy-1,4-benzoxazin oder N-Methyl-6-hydroxy-1,4-benzoxazin ausgewählt ist.

4. Zusammensetzung zum Färben nach einem der Ansprüche 1 bis 3, dadurch gekennzeichnet, daß sie mindestens 2-(β-Hydroxyethyl)-p-phenylendiamin als Farbstoffvorprodukt der Oxidationsfarbe der Formel (I) und mindestens 6-Hydroxy-1,4-benzoxazin oder N-Methyl-6-hydroxy-1,4-benzoxazin als Kuppler der Formel (II) oder die Additionssalze dieser Verbindungen mit einer Säure enthält.

5. Zusammensetzung zum Färben nach einem der Ansprüche 1 bis 3, dadurch gekennzeichnet, daß sie mindestens 2-(β-Hydroxyethyloxy)-p-phenylendiamin als Farbstoffvorprodukt der Oxidationsfarbe der Formel (I) und mindestens 6-Hydroxy-1,4-benzoxazin oder N-Methyl-6-hydroxy-1,4-benzoxazin als Kuppler der Formel (II) oder die Additionssalze dieser Verbindungen mit einer Säure enthält.

6. Zusammensetzung zum Färben nach Anspruch 1, dadurch gekennzeichnet, daß sie mindestens 2-(β-Hydroxyethyl)-p-phenylendiamin als Farbstoffvorprodukt des Oxidationsfarbstoffs der Formel (I), 6-Hydroxy-1,4-benzoxazin als Kuppler und 6-Hydroxyindol als zusätzlichen Farbstoff oder die Additionssalze dieser Verbindungen mit einer Säure enthält.

7. Zusammensetzung zum Färben nach einem der Ansprüche 1 bis 6, dadurch gekennzeichnet, daß die Additionssalze mit einer Säure unter Hydrochloriden, Sulfaten, Hydrobromiden und Tartraten ausgewählt sind.

8. Zusammensetzung zum Färben nach einem der Ansprüche 1 bis 7, dadurch gekennzeichnet, daß das p-Phenylendiamin der Formel (I) oder seine Salze in einer Konzentration von 0,01 bis 10 Gew.-% und vorzugsweise von 0,05 bis 5 Gew.-%, bezogen auf das Gesamtgewicht der Zusammensetzung, und der Kuppler der Formel (II) oder seine Salze in einer Konzentration von 0,05 bis 5 Gew.-% und vorzugsweise im Bereich von 0,01 bis 3 Gew.-%, bezogen auf das Gesamtgewicht der Zusammensetzung, vorliegen.

9. Zusammensetzung zum Färben nach einem der Ansprüche 1 bis 8, die anwendungsfertig ist und die dadurch gekennzeichnet ist, daß sie ferner ein Oxidationsmittel enthält und einen pH-Wert von 3 bis 11 aufweist.

10. Verfahren zum Färben von Keratinfasern und insbesondere von menschlichen Keratinfasern, wie dem Haar, dadurch gekennzeichnet, daß es darin besteht, auf die Fasern eine Zusammensetzung zum Färben (A) nach einem der Ansprüche 1 bis 9 aufzutragen und die Farbe im alkalischen, neutralen oder sauren Medium mit einem Oxidationsmittel zu entwickeln, das dieser Zusammensetzung (A) bei der Anwendung zugesetzt wird oder das in einer Zusammensetzung (B) vorliegt, die gleichzeitig oder getrennt davon anschließend aufgetragen wird.

11. Vorrichtung mit mehreren Abteilungen oder "Kit" zum Färben von Keratinfasern und insbesondere menschlichen Keratinfasern, wie dem Haar, dadurch gekennzeichnet, daß sie mindestens zwei Abteilungen aufweist, wobei eine Abteilung eine Zusammensetzung (A) nach den Ansprüchen 1 bis 8 und die andere eine Zusammensetzung (B) enthält, die in einem zum Färben geeigneten Medium ein Oxidationsmittel enthält.

12. Verwendung einer Zusammensetzung zum Färben nach den Ansprüchen 1 bis 9 oder einer Vorrichtung zum Färben oder eines "Kits" mit mehreren Abteilungen nach Anspruch 11 zum Färben von menschlichen Keratinfasern, wie dem Haar.
